# EUROPEAN PATENT APPLICATION

(11) **EP 4 537 797 A1**
(43) Date of publication of application: **16.04.2025**
(21) Application number: 24789021.3
(22) Date of filing: 11.04.2024
(51) Int. Cl.: A61F 5/11, A61F 13/00

(54) **NAIL CORRECTION BAND**

(30) Priority: 11.04.2023 KR 20230047368
(71) Applicant: Freshfoot Leessam Co., Ltd., Seoul 04790 (KR)
(72) Inventor: BAE, Seong Gyu, Namyangju-si Gyeonggi- do 12218 (JP)
(74) Representative: Impuls legal PartG mbB
(86) International application number: PCT/KR2024/004786
(87) International publication number: WO 2024/215079

(57) **Abstract**

The disclosed nail correction band comprises: a first adhesive portion (10) formed on one edge and having a first adhesive surface (11); a second adhesive portion (20) formed on the other edge and having a second adhesive surface (21); a correction portion (30) included between the first adhesive portion (10) and the second adhesive portion (20); and an elastic unit (60) arranged over the entire first adhesive portion (10), the correction portion (30), and the second adhesive portion (20); wherein the first adhesive portion (10) further comprises a first adhesive extension portion (12) extending outwardly, and the second adhesive portion (20) further comprises a second adhesive extension portion (22) extending outwardly, and the elastic units (60) are provided in two or more numbers, and each elastic unit (60) is configured with a different length.

## Description

### [Technical Field]

The present invention relates to a nail correction band for correction of ingrown nail, and more particularly to an ingrown nail correction band for correction of ingrown fingernail or toenail in which the edges of fingernail or toenail grow into the surrounding tissues.

### [Background Art]

Ingrown fingernail or toenail are a phenomenon in which the edges of fingernail or toenail (hereinafter referred to as "nail") grow into the tissues of the human body. These ingrown fingernail or toenail grow with their edges digging into the flesh, causing inflammation and pain in the fingers or toes, and also damaging the appearance of the fingernail or toenail to the extent that it can interfere with social life.

In the past, surgical operations were performed to form this phenomenon into a normal shape, but surgical approaches took a long time to recover and were painful.

Many devices are being developed to replace these surgical operations.

The principle of these devices is that they are made of elastic materials, and they use tensile force as a force that pulls objects that are spatially separated from each other to straighten the edges of the fingernail that have been attached for a long time, and these devices are removed over time.

In particular, as in the present invention, the band type attached to the nail is used by attaching it to the edge of the nail and then spreading the nail by tensile force, and as the attached nail grows over time, it is cut off together with the band attached to the nail, or the band attached to the nail is removed.

Therefore, as much as tensile force is used, sufficient tension must be applied to re-ingrown nail, and pain caused by excessive tensile force must not occur.

In addition, since devices such as orthodontic bands must be kept attached to the nail for a long time, it is desirable to prevent discomfort or secondary pain caused by such orthodontic bands when living with the orthodontic bands attached to the nail.

In relation to devices for replacing surgical operations for ingrown nail, a nail corrector comprising "a plate-shaped elastic piece having one end extending toward one of the two lateral ends of the nail and the other end positioned on the inside of the nail; a hook portion that is bent from one end of the elastic piece toward the nail and is inserted into one of the two lateral ends of the nail; and a plate-shaped contact portion formed at the other end of the elastic piece and at least a portion of which is interposed between the elastic piece and the nail to elastically contact the nail, and made of a shape memory alloy" has been introduced, but this has a problem in that it may cause inconvenience in daily life because it includes a metal material.

As another example, "in an ingrown toenail corrector that correctly corrects an ingrown toenail into a normal toenail by mounting an elastic plate spring on the toenail, the plate spring is composed of an upper plate (10), a lower plate (20), and a fixing part that connects one end of the upper plate (10) and the lower plate (20), and the fixing part is installed at each of the left and right ends as a left fixing part (90) and a right fixing part (30), and the right fixing part (30) is composed of a body (31) installed on the upper plate (10) and a fixing piece (33) connected to the body (31) by a hinge (32), so that after the upper plate (10) and the lower plate (20) overlap with the upper surface of the body (31), the fixing piece (33) is rotated counterclockwise to the left about the hinge so that the lower end of the fixing piece (33) presses the upper surface of the upper plate (10), thereby An "ingrown toenail corrector" has been introduced, characterized by the overlapping portion of the upper plate (10) and lower plate (20) being fixed by the right fixing part (30), but there is a problem that it is inconvenient to wear and live with during the correction process.

As another example, a "nail correction device (10) comprising a first adhesive element (12) and a second adhesive element (14), an extension element (16) that acts elastically between the first and second adhesive elements (12, 14), wherein the first adhesive element (12) and the second adhesive element (14) are arranged at a predetermined distance from each other and are connected to each other via the extension element (16), and the first and second adhesive elements (12, 14) can be respectively fixed to an area of the nail plate via the adhesive connection, and the first adhesive element (12), the second adhesive element (14) and the extension element (16) form an inseparable unit and are designed as a single piece, and the extension element (16) acts elastically in the longitudinal direction and functions as a traction element" has been disclosed, but there is a limitation that each size must be manufactured according to the size of the human body part.

As another example, an ingrown nail correction strip characterized in that it comprises an integrated product including "a band-shaped adhesive sticker that serves as a base material; an elastic correction tip that is adhesively fixed to the center of one side of the band-shaped adhesive sticker and is used to correct an ingrown nail by being adhesively fixed to an ingrown nail; a release paper that is positioned so as to be positioned on the upper surface of the correction tip and is adhesively fixed to the band-shaped adhesive sticker, and has a perforation formed therein so that the correction tip adhesively fixed on the band-shaped adhesive sticker is exposed; a protective sticker that is adhesively fixed to the upper surface of the release paper and covers and protects the correction tip" has been disclosed, but the adhesive strength is low, and thus, there is a hassle of requiring additional work to increase the adhesive strength, as in the case of existing types.

### [Disclosure]

### [Technical Problem]

The present invention aims to provide a correction band that can replace a surgical procedure that involves time investment and pain for correcting an ingrown nail.

In addition, the present invention aims to provide a correction band that is cost-saving through a simple configuration compared to other prior technologies.

In addition, the present invention aims to provide a correction band that users can easily attach themselves.

In addition, the present invention aims to provide a correction band that can overcome differences in nail size as a correction band that has universality despite the differences in nail size among users.

In addition, the present invention aims to provide a correction band that does not cause a foreign feeling due to the attachment of the correction band in daily life during the correction process.

### [Technical Solution]

A nail correction band according to the present invention comprises: a first adhesive portion (10) formed on one edge and having a first adhesive surface (11); a second adhesive portion (20) formed on the other edge and having a second adhesive surface (21); a correction portion (30) included between the first adhesive portion (10) and the second adhesive portion (20); and an elastic unit (60) arranged over the entire first adhesive portion (10), the correction portion (30), and the second adhesive portion (20); wherein the first adhesive portion (10) further comprises a first adhesive extension portion (12) extending outwardly, and the second adhesive portion (20) further comprises a second adhesive extension portion (22) extending outwardly, and the elastic units (60) are provided in two or more, and each elastic unit (60) is configured with a different length.

In the nail correction band according to the present invention, the first adhesive surface (11) and the second adhesive surface (21) each include an adhesive groove (13, 23), and the adhesive groove (13, 23) includes a grid structure of one or more cavities (14, 24), and the cavity (14, 24) further includes a dovetail structure (15, 25) on the inside to further increase the adhesive strength.

In the nail correction band according to the present invention, the correction portion (30) includes a fastening part (40) for reducing the length of the correction portion (30), and the fastening part (40) is configured to have a thickness thinner than the thickness of the correction portion (30) and includes a catch (41).

In the nail correction band according to the present invention, a handle (50) is provided on the upper surface (1b) of at least one of the first adhesive portion (10), the second adhesive portion (20), and the correction portion (30).

### [Advantageous Effects]

The band of the present invention has a corrective effect as a simple device rather than a painful surgical procedure for correcting ingrown toenail.

In addition, it has a cost-saving effect due to its relatively simple configuration compared to other existing devices.

In addition, it has an effect that users can easily attach and use it themselves.

In addition, it has an effect that provides versatility to people with different nail sizes depending on their body type.

In addition, it has an effect that provides convenience in life because it does not interfere with daily life after attachment during the correction process.

### [Description of drawings]

Fig.1 is a drawing showing a frontal configuration of a nail correction band according to the present invention.
Fig.2 is a drawing showing an example of implementing a nail correction band according to the present invention.
Fig.3 is a drawing showing in detail an adhesive groove of a nail correction band according to the present invention.
Fig.4 is a drawing showing a cross-sectional configuration of a nail correction band according to the present invention.
Fig.5 is a drawing showing an example of a nail correction band according to the present invention in which a handle is formed at the end of an adhesive portion.
Fig.6 is a drawing showing another example of a nail correction band according to the present invention.
Fig.7 is a drawing showing a side view of the nail correction band of Figure 1.
Fig.8 is a drawing showing a modified example of Figure 7.
Fig.9 is a drawing showing a cross-section of Figure 3.

### [Mode for Invention]

Hereinafter, if it is judged that a detailed description of related known technology may unnecessarily obscure the gist of the present invention, the detailed description will be omitted. In addition, the numbers used in the description process of this specification are merely identifiers to distinguish one component from another.

In addition, the terms used in this specification and claims should not be interpreted as limited to their dictionary meanings, and should be interpreted as meanings and concepts that conform to the technical idea of the present invention based on the principle that the inventor can appropriately define the concept of the term in order to explain his or her invention in the best way.

Therefore, the embodiments described in this specification and the configurations illustrated in the drawings are only preferred embodiments of the present invention, and do not express all of the technical ideas of the present invention, so it should be understood that various equivalents and modified examples may exist that can replace them at the time of this application.

The preferred embodiments of the present invention will be described in more detail, but the technical parts that are already well-known will be omitted or compressed for the sake of brevity of the description. The nail used in the present invention refers to a fingernail or toenail, and can mainly correspond to a thumb, but can be applied to either a fingernail or toenail.

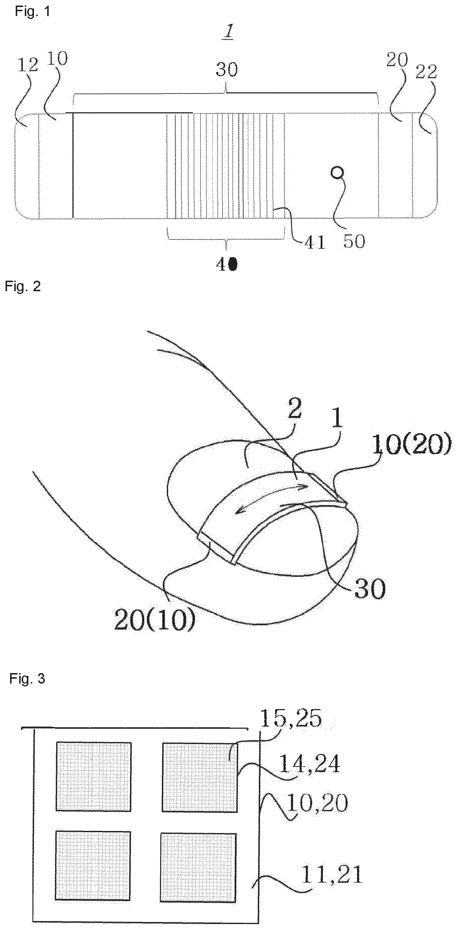

The present invention is to attach to a fingernail and provide tensile force to straighten an ingrown nail, and as shown in FIG. 1, a correction band (1) for straightening an ingrown nail includes a first adhesive part (10) on one side edge, a second adhesive part (20) on the other side edge, and a correction portion (30) in the center.

As shown in FIG. 1, the correction band (1) can be configured as an integral part.

Preferably, it can be configured as an integral part as a single plate-shaped structure in a band-like shape.

The correction band (1) can be configured as a single plate-shaped structure in a band-like shape, and it is preferable that it is relatively thin, has a constant length, and is configured in a band-like shape. The material of the correction band (1) may be the same material for the first adhesive portion (10), the second adhesive portion (20), and the correction portion (30), or may be different materials.

The material of the correction band (1) may be composed of any one of thermoplastic polyurethane, PVC, rubber, or synthetic resin that has elasticity to be attached to the nail (2) and generate tensile force, and is not limited to the materials presented above.

The overall shape of the correction band (1) may have an angular shape or a rounded shape at the corners, and there is no particular limitation on the shape of the corners.

In addition, the vertex of the correction band (1) may have an angular shape or a rounded shape, and there is no particular limitation on the shape of the vertex.

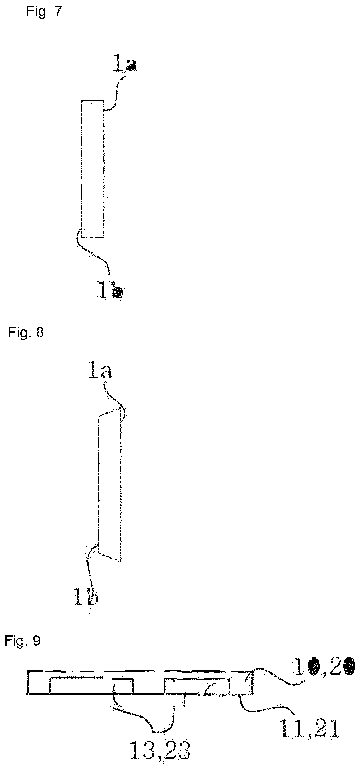

As shown in Fig. 7, the correction band (1) may have a contact surface (1a) and an upper surface (1b) that contact the nail with the same length, and the cross section of the side may be a rectangular shape. Or, in order to reduce the contact surface of stockings or socks, etc. while the correction band (1) is attached to the nail (2), the correction band (1) may have a length such that the upper surface (1b) is narrower than the contact surface (1a) that contacts the nail (2), as shown in Fig. 8, and the cross-section of the side may be in the shape of a diamond.

Meanwhile, the first adhesive portion (10) or the second adhesive portion (20) may be configured to have a wider shape than the correction portion (30) to increase the contact surface.

When the correction band (1) is attached to the center of the nail (2) and the correction band (1) is removed after correction, or when the correction band (1) is attached to the end of the nail (2) and the nail is cut off while the correction band is attached to the nail when it has grown, or when the correction band (1) is attached to the nail, the width of the correction band (10) is preferably 3 mm to 7 mm for convenience in daily life. As shown in FIG. 2, the portion of the first adhesive portion (10) or the second adhesive portion (20) of the present invention is bonded to the edge of the nail (2). More specifically, as shown in FIG. 2, the first adhesive surface (11), which is the contact surface (1a) of the first adhesive portion (10) that contacts the nail (2), and the second adhesive surface (21), which is the contact surface (1a) of the second adhesive portion (20) that contacts the nail (2) can be bonded to the edge of the nail (2).

An adhesive is used to bond the first adhesive surface (11) or the second adhesive surface (21) to the nail (2).

At this time, there is no particular limitation on the adhesive for bonding the first and second adhesive surfaces (11, 21) of the correction band (1) and the nail (2), but it is preferable that it be a material that does not react with the material of the correction band (1). In this way, when using the correction band (1), adhesive can be directly applied to the first adhesive surface (11) or the second adhesive surface (21) to adhere to the edge of the nail (2).

Meanwhile, when adhesive is applied in advance to the first adhesive surface (11) or the second adhesive surface (21), a release sheet can be attached to the contact surface (1a) of the correction band (1), and when using, the release sheet can be removed and adhered to the nail (2).

The first adhesive surface (11) of the first adhesive portion (10) can include an adhesive groove (13) to increase adhesive strength, and the second adhesive surface (21) of the second adhesive portion (20) can include an adhesive groove (23) to increase adhesive strength. The above adhesive grooves (13, 23) can further increase the adhesive strength by adding frictional force as well as adhesive force when attached to the edge of the nail (2) so that the correction band (1) does not fall off during daily life after being attached to the nail (2).

As shown in FIGS. 3 and 9, the adhesive grooves (13, 23) can be configured as a grid structure of one or more cavities (Cavities, 14, 24) such as squares or rectangles.

At this time, each cavity can further include a dovetail structure (Dovetail, 15, 25) on the inside to further increase the adhesive strength.

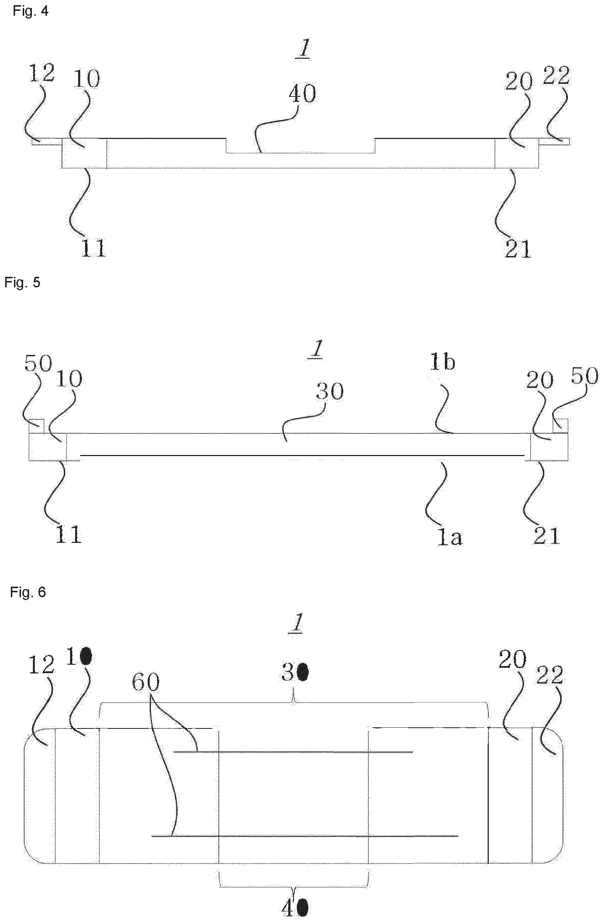

As shown in FIGS. 1 and 4, the first adhesive portion (10) can further include a first adhesive extension portion (12) that extends outward.

In addition, the second adhesive portion (20) can further include a second adhesive extension portion (22) that extends outward. In the case of users with severe ingrown toenail (2), there are cases where the upper part of the toenail is severely curved. In such cases, the toenail is usually trimmed and then the correction band (1) is attached. However, even then, the first adhesive surface (11) and/or the second adhesive surface (21) of the correction band (1) may not adhere well to the edge of the toenail (2).

To overcome the above cases, it may be more efficient to maintain the state of adhesion to the toenail (2) by lifting the inner side of the toenail and adhering the first adhesive extension part (12) and/or the second adhesive extension part (22) inward.

Therefore, since the first adhesive extension part (12) and/or the second adhesive extension part (22) are adhered to the inner side of the toenail (2), the correction band (1) can be prevented from coming off during daily life after the toenail is adhered. The above first adhesive extension part (12) and the second adhesive extension part (22) are preferably configured with a thickness thinner than the thickness of the correction band (1) because they are adhered to the inner side of the nail (2).

Meanwhile, the correction portion (30) forms a space between the first adhesive part (10) and the second adhesive part (20). The correction portion (30) is not adhered to the nail (2), but has a function of stretching the nail (2) by pulling the edge of the nail (2) attached to the first adhesive part (10) and the second adhesive part (20) due to the tensile force of its material.

Therefore, the first adhesive part (10) and the second adhesive part (20) and the correction portion (30) may be formed of the same material, but may be formed of a different material from the first adhesive part (10) and the second adhesive part (20) in order to increase the tensile force of the correction portion (30). As shown in FIGS. 1 and 4, the correction portion (30) may include a fastening part (40) for reducing the length of the correction band (1).

The width or length of the nail (2) varies greatly depending on race, body type, or age. Since the length of the edge bonding position varies depending on the difference in the width of the nail (2), a problem may occur in which the length of the correction band (1) varies.

Therefore, if the length of the correction band (1) is standardized to a certain length, the components of the first adhesive part (10), the second adhesive part (20), and the correction portion (30) may be improperly bonded to the nail (2) or the effect of straightening through tensile force may be significantly reduced.

To solve this problem, some of the bands among the conventional products that are bonded to the nail (2) for correction are provided as a set with the total length of the band being different.

To solve the above problem, the present invention includes a fastening part (40) inside the correction portion (30). The above-mentioned fastening part (40) can be formed with the same thickness as the correction band (1).

Preferably, the above-mentioned fastening part (40) can be configured with a thickness thinner than the correction band (1) as shown in FIG. 4.

The above-mentioned fastening part (40) can be bonded with a little extra length after the first adhesive part (10) and the second adhesive part (20) are bonded to the edge of the nail (2) or overlapped to further increase the traction force, thereby increasing the tensile strength.

In addition, in order to prevent the fastening part (40) from being detached during daily life during the correction process after the correction band (1) is bonded to the nail (2), the above-mentioned fastening part (40) can include a catch (41) as shown in FIG. 1.

The catch (41) can be included in the upper surface (1b) of the fastening part (40) and/or the contact surface (1a) that contacts the nail (2).

As shown in FIG. 1, one or more handles (50) may be included on the upper surface (1b) of the correction band (1) so that the user can accurately adhere the correction band (1) to the edge of the nail (2).

The position of the handle (50) is not particularly limited to the positions of the first adhesive portion (10), the second adhesive portion (20), and the correction portion (30), but is preferably included in the first adhesive portion (10) or the second adhesive portion (20).

More preferably, as shown in FIG. 5, it is preferably included in the upper surface (1b) of the first adhesive portion (10) or the second adhesive portion (20). In addition, it may be included on one or both sides of the first adhesive portion (10) or the second adhesive portion (20).

As shown in FIG. 6, the correction band (1) may include one or more elastic units (60) on the upper surface (1b). The elastic unit (60) is intended to increase the tensile strength of the correction band (10) and may be composed of an elastic body.

The elastic unit (60) may be characterized by being arranged throughout the first adhesive portion (10), the correction portion (30), and the second adhesive portion (20).

Each elastic unit (60) is configured with the same length so that the portions including each elastic unit (60) can equally enhance the tensile force, or is configured with different lengths so that the portions including each elastic unit (60) can have different tensile forces.

For example, as shown in FIG. 6, if the upper elastic unit (60) is configured with a short length and the lower elastic unit (60) is configured with a relatively long length, the tensile force can be applied more to a portion of the nail that requires more traction when attaching the nail.

As described above, the specific description of the present invention has been made by way of examples, but since the above-described examples have only described preferred examples of the present invention, it should not be understood that the present invention is limited to the above-described examples, and the scope of the rights of the present invention should be understood by the claims described below and their equivalent concepts.

## Claims

1. A nail correction band, comprising:
a first adhesive portion (10) formed on one edge and having a first adhesive surface (11);
a second adhesive portion (20) formed on the other edge and having a second adhesive surface (21);
a correction portion (30) arranged between the first adhesive portion (10) and the second adhesive portion (20); and
an elastic unit (60) arranged over the entire first adhesive portion (10), the correction portion (30), and the second adhesive portion (20);
wherein the first adhesive portion (10) further comprises a first adhesive extension portion (12) extending outward, the second adhesive portion (20) further comprises a second adhesive extension portion (22) extending outward, and the elastic units (60) are provided in two or more, and each elastic unit (60) is configured with a different length.

2. The nail correction band of claim 1, wherein the first adhesive surface (11) and the second adhesive surface (21) each comprise an adhesive groove (13, 23), the adhesive groove (13, 23) comprises a lattice structure of one or more cavities (14, 24), and the cavity (14, 24) further comprises a dovetail structure (15, 25) on the inside to further increase the adhesive strength.

3. The nail correction band of claim 1, wherein
the correction portion (30) comprises a fastening part (40) for reducing the length of the correction portion (30), and
the fastening part (40) is configured to have a thickness thinner than the thickness of the correction portion (30) and includes a catch (41).

4. The nail correction band of claim 1, further comprises a handle (50) provided on the upper surface (1b) of at least one of the first adhesive portion (10), the second adhesive portion (20), and the correction portion (30).
